(19) Europäisches Patentamt
European Patent Office
Office européen des brevets

(11) EP 1 083 184 A2

(12) # EUROPÄISCHE PATENTANMELDUNG

(43) Veröffentlichungstag:
**14.03.2001 Patentblatt 2001/11**

(51) Int. Cl.[7]: **C08F 2/24**, A61K 7/06

(21) Anmeldenummer: **00118465.4**

(22) Anmeldetag: **25.08.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **07.09.1999 DE 19942565**
**28.10.1999 DE 19951877**

(71) Anmelder:
**BASF AKTIENGESELLSCHAFT**
**67056 Ludwigshafen (DE)**

(72) Erfinder:
- **Witteler, Helmut, Dr.**
  **67259 Beindersheim (DE)**
- **Sanner, Axel, Dr.**
  **67227 Frankenthal (DE)**
- **Hössel, Peter, Dr.**
  **67105 Schifferstadt (DE)**

(54) **Silikonhaltige Polymerisate, deren Herstellung und Verwendung**

(57) Die vorliegende Erfindung betrifft ein wasserlösliches oder redispergierbares Polymerisat, erhältlich durch radikalische Polymerisation von ethylenisch ungesättigten Monomeren A in Gegenwart wenigstens eines polyalkylenoxid-haltigen Silikonderivates B, dadurch gekennzeichnet, daß für das Produkt aus Ionenaustauscherequivalent IEE und K-Wert des Polymers gilt

$$\text{K-Wert} \times \text{IEE} \geq 150 \text{ mmol g}^{-1},$$

und das IEE > 2.7 mmol $g^{-1}$ ist und, daß das IEE auf Carbonsäuregruppen oder Carboxylatgruppen beruht, ein Verfahren zu seiner Herstellung sowie die Verwendung als Zusatzstoff für kosmetische, dermatologische, hygienische und/oder pharmazeutische Zubereitungen.

EP 1 083 184 A2

## Beschreibung

Die vorliegende Erfindung betrifft silikonhaltige Polymerisate, deren Herstellung und Verwendung, sowie kosmetische, dermatologische, hygienische und/oder pharmazeutische Zubereitungen, die diese Polymere enthalten.

Polymere mit filmbildenden Eigenschaften werden in der Kosmetik als Filmbildner für kosmetische, dermatologische, hygienische und/oder pharmazeutische Formulierungen verwendet und eignen sich insbesondere als Zusatzstoffe für Haar- und Hautkosmetika.

In den kosmetischen und dermatologischen Zubereitungen für die Haut können die erfindungsgemäßen Polymere besondere Wirkung entfalten. Die Polymere können unter anderem zur Feuchthaltung und Konditionierung der Haut und zur Verbesserung des Hautgefühls beitragen. Die Haut wird glatter und geschmeidiger.

Bevorzugt werden die Polymere zur Festigung, Strukturverbesserung und Formgebung der Haare verwendet. Sie erhöhen die Kämmbarkeit und verbessern den Griff des Haares. Diese Haarbehandlungsmittel enthalten im Allgemeinen eine Lösung des Filmbildners in einem Alkohol oder einem Gemisch aus Alkohol und Wasser.

Eine Anforderung an Haarbehandlungsmittel ist es, dem Haar unter anderem Glanz, Flexibilität und natürlichen, angenehmen Griff zu verleihen.

Es ist bekannt Vinyllactam-Homo- und Copolymere oder carboxylatgruppenhaltige Polymere einzusetzen. Das gewünschte Eigenschaftsprofil wie starke Festigung bei hoher Luftfeuchtigkeit, Elastizität, Auswaschbarkeit vom Haar und Verträglichkeit mit den übrigen Formulierungskomponenten wird durch Copolymerisation einer Kombination von hydrophoben, elastifizierenden und Carboxylgruppen enthaltenden Monomeren erzielt.

Der Griff, der mit diesen Polymeren gefestigten Frisuren ist allerdings unangenehm stumpf und unnatürlich. Die Zugabe von Weichmachern verbessert zwar den Griff solcher Frisuren, reduziert aber gleichzeitig die Festigungswirkung.

Häufig werden Polysiloxane eingesetzt, die aber mit den polaren Polymeren nicht verträglich sind und oft weitere Zusätze verlangen, um überhaupt formuliert werden zu können. Entmischungen können sowohl während der Lagerung der Formulierung als auch während des Gebrauchs zu Problemen führen.

EP-A 670 342 beschreibt die Verwendung alkoxylierter Silikone in Haarpflegemitteln. Die Verwendung von Polymerisaten aus ungesättigten Verbindungen in Haarpflegemitteln wird nicht offenbart. Die Verwendung alkoxylierter Silikone als Zusatz zu handelsüblichen Haarfestiger-Polymeren verbessert zwar deren Griff, führt aber gleichzeitig zu verringerter Festigungswirkung.

Die europäischen Patentschriften EP-A 412 704 und EP-A 412 707 beschreiben Polysiloxangruppen in Form von Makromonomeren mit Molmassen von 1000 bis 50 000, die mit üblichen hydrophoben und hydrophilen Monomeren polymerisiert werden. Die Synthese dieser Monomeren ist außerordentlich aufwendig. Aus den Polymeren können nicht umgesetzte Makromonomere und deren unreaktive Verunreinigungen aufgrund ihres hohen Molekulargewichtes kaum abgetrennt werden. Sie stellen ein toxikologisches und allergenes Risiko dar. Darüber hinaus sind die erhaltenen Copolymeren, um eine gute Wirkung zu erzielen, oft nur in Kombination mit weiteren Polymeren, Carriern und weiteren Hilfsstoffen zu formulieren, wie die o.g. Patentschriften lehren.

DE-A 19 73 1529 beschreibt Silkontensid-Acrylat-Copolymere. Die dort genannten Beispiele zeigen eine Limitierung des Anteils an Monomeren mit freien Carboxylatgruppen in der Reaktionsmischung.

In EP-A 614 924 werden Copolymere von Silikonmakromonomeren beschrieben. Bei mangelnder Verträglichkeit des Silikonmakromonomers mit den anderen Monomeren während der Synthese tritt aber unerwünschte Koagulatbildung auf.

In DE-A 29 17 504 und in EP-A 832 638 werden Polymerisate mit hohem Anteil an Carboxylatgruppen beschrieben. Bei solchen Polymerisaten ist die Löslichkeit in Alkohol/Kohlenwasserstoff-Treibmitteln stark herabsetzt.

Die aus dem Stand der Technik bekannten Herstellungsverfahren von Copolymeren aus ethylenisch ungesättigten Monomeren in Emulsionsverfahren weisen ein Reihe von Nachteilen auf. Wird beispielsweise versucht, ein hohes Molekulargewicht (gekennzeichnet etwa durch einen K-Wert > 50) einzustellen, kommt es zur Koagulation der Emulsion. Die Polymerisation muß abgebrochen werden, ohne daß das gewünschte Produkt erhalten werden kann.

Auch wenn in Gegenwart von Silikonen mit niedrigem HLB-Wert polymerisiert wird, kommt es leicht zur Koagulation; ebenso, wenn in Gegenwart von Silikonen größere Mengen von ethylenisch ungesättigten Carbonsäuren (typischerweise Acrylsäure, Methacrylsäure und Crotonsäure in Mengen, die dem resultierenden Polymer ein IEE größer als 4 mmol $g^{-1}$ geben sollen) zur Polymerisation eingestzt werden.

Der vorliegenden Erfindung lag daher die Aufgabe zugrunde neuartige Polymere für kosmetische, hygienische und/oder pharmazeutische Formulierungen ohne die geschilderten Nachteile bereitzustellen. Die erfindungsgemäßen Polymere sollten filmbildend, löslich und nicht klebrig sein, und nach Applikation den sensorischen Eindruck geringer Oberflächenrauhigkeit bzw. großer Glätte liefern, um sie bei gleichzeitiger Löslichkeit oder Redispergierbarkeit in Wasser und Löslichkeit in Alkohol/KohlenwasserstoffTreibmitteln in kosmetischen, dermatologischen, hygienischen und/oder pharmazeutischen Formulierungen vorteilhaft einsetzen zu können..

Zur Herstellung des Polymers sollte ein wirtschaftliches Verfahren angewandt werden, daß nicht zur Bildung

von unbrauchbaren Nebenprodukten (z. B. Koagulat bei der Emulsionspolymerisation) führt.

Für haarkosmetische Formulierungen bestand ferner die Aufgabe, ein Polymer zur Verfügung zu stellen, mit dem sich gute Kämmbarkeit, starke Festigung und guter Griff realisieren lassen.

Überraschenderweise wurde nun gefunden, dass diese Aufgabe gelöst wird durch ein wasserlösliches oder redispergierbares Polymerisat, erhältlich durch radikalische Polymerisation von ethylenisch ungesättigten Monomeren A in Gegenwart von alkylenoxid-haltiger Silikonderivaten B mit geringem hydrophilen Charkter, dadurch gekennzeichnet, daß für das Produkt aus Ionennaustauscheräquivalent IEE und K-Wert des Polymers gilt, K-Wert x IEE ≥ 150 mmol $g^{-1}$ und IEE > 2,7 mmol $g^{-1}$, und daß das IEE auf Carbonsäuregruppen oder Carboxylatgruppen beruht. Die Monomere A bestehen bevorzugt sowohl aus ethylenisch ungesättigten Monomere M als auch aus ethylenisch ungesättigten Monomere C mit freien Carboxylatgruppen.

Die vorteilhaften Eigenschaften der erfindungsgemäßen Polymere führen in kosmetischen Formulierungen zu der erwünschten Eigenschaftskombination filmbildend, wasserlöslich, löslich in üblichen Treibgas/Ethanol-Mischungen, nicht klebrig und hohe Glätte nach der Applikation. Diese Eigenschaften sind von Polymeren mit einem IEE ≥ 3.5 mmol $g^{-1}$ aus dem Stand der Technik insbesondere für die Haarkosmetik nicht bekannt und auch nicht zu erwarten, da hohe Festigung als Folge hoher mechanischer Festigkeit von linearen Polymeren, die leicht Verhakungsnetzwerke bilden können, eher erwartet wird, als von kammförmigen Pfropfcopolymeren, wie sie bei der Polymerisation von ethylenisch ungesättigten Monomeren A in Gegenwart alkylenoxid-haltiger Silikonderivate B gebildet werden. Außerdem ist bei hohen Molekulargewichten, wie sie durch die oben gekennzeichneten K-Werte erhalten werden, für den Fachmann nicht mit guter Kämmbarkeit zu rechnen. Auch die erhöhte Konzentration von Carboxylatgruppen ergibt bei Polymeren grundsätzlich, auch wenn sie zu einer größeren mechanischen Steifigkeit führt, eine niedrigere mechanische Festigkeit. Insofern war von der erfindungsgemäßen Kombination aus hohem Carboxylatgruppenanteil und hohem Molekulargewicht kein Anwendungsvorteil für Haarfestigerformulierungen zu erwarten.

Sind die Silikonderivate B nicht während der Polymerisation zugegen, sondern werden nach der Polymerisation eingemischt, so erhält man in der Regel sehr weiche klebrige Filme, die für die erfindungsgemäßen Anwendungen in der Haut- und Haarkosmetik ungeeignet sind. Dies deutet darauf hin, daß es während der Polymerisation eventuell zu einer Pfropfung der Polymerisate auf die Silikonverbindungen kommen kann, und dies zu den guten Filmeigenschaften wie Klebfreiheit, hohe Oberflächenglätte und Härte beiträgt. Es sind jedoch auch andere Mechanismen als Pfropfung vorstellbar, durch die die erfindungsgemäßen Polymere zu ihren vorteilhaften Eigenschaften kommen.

Ein weiterer Gegenstand der Erfindung ist die Verwendung der erfindungsgemäßen Polymere als Zusatzstoffe für kosmetische, dermatologische, hygienische und/oder pharmazeutische Zubereitungen sowie kosmetische, dermatologische, hygienische und/oder pharmazeutische Zubereitungen enthaltend die erfindungsgemäßen Polymere, deren Herstellung nach den üblichen, dem Fachmann geläufigen Regeln erfolgt.

Die Bezeichnung Ionenaustauscher-Äquivalent (Abkürzung IEE) bezeichnet hier den Gehalt von ionischen Gruppen in einem Polymer. Beispielsweise ist IEE für reine Polymethacrylsäure 10 mmol $g^{-1}$.

Mit "redispergierbar" im Sinne der Erfindung sind Polymere gemeint, die im Kontakt mit Wasser in einem endlichen Zeitraum ein Fluid bilden, das ohne optische Hilfsmittel mit dem Auge keine festen Partikeln erkennen läßt. Zur Überprüfung, ob ein Polymer in Wasser redispergierbar ist, werden 100 mg des Polymers in Form eines 100 µm dicken Films in 100 mL Wasser (20°C) gegeben und auf einem handelsüblichen Schütteltisch für 24 Stunden geschüttelt. Wenn nach dem Schütteln keine festen Partikeln mehr erkennbar sind, das Fluid aber eine Trübung besitzt, ist das Polymer in Wasser redispergierbar; ohne Trübung wird es als wasserlöslich bezeichnet.

Der HLB-Werte sind ein Maß für die Wasser- bzw. Öl-Löslichkeit von vorwiegend nichtionischen Tensiden. Substanzen mit HLB < 10 sind meist gute W/O-Emulgatoren, während Substanzen mit höherem HLB-Wert als O/W-Emulgatoren wirken. Substanzen mit HLB-Werten kleiner als 10 bis 13 bilden in Wasser keine klare Lösung. Eine Methode zur experimentellen Bestimmung von HLB-Werten ist beschrieben in Kirk-Othmer, Encyclopedia of Chemical Technology, 3rd Ed, Wiley., New York 1965, Band 8, Seite 132.

Die erfindungsgemäßen Polymerisate weisen bevorzugt einen K-Wert (auch Fikentscher-Konstante, vgl. H.-G. Elias, Makromoleküle, Bd. 1, Hüthig & Wepf, Heidelberg 1990, S. 98 f.) von größer 45 auf, gemessen bei einer Lösungskonzentration des Polymers von 0,5 bis 10 Gew.-%, bevorzugt 0,5 bis 1,5 Gew.-% in Ethanol (T = 23°C).

Als geeignete polymerisierbare Monomere A werden bevorzugt Polymere eingesetzt, die sich durch die folgenden Merkmale auszeichnen: K-Wert des Polymers ≥ 45, IEE des Polymers ≥ 3.5 mmol $g^{-1}$ und gegebenenfalls HLB des Silikons ≤ 13. Erfindungsgemäß sind ferner Polymere für die gilt

$$\text{IEE} \times \text{K-Wert} \geq 150 \text{ mmol g}^{-1}$$

und hier insbesondere

$$\text{IEE} \times \text{K-Wert / HLB-Wert} \geq 12 \text{ mmol g}^{-1}.$$

Besonders bevorzugt sind Polymere, für die gilt: K-Wert des Polymers ≥ 50, IEE des Polymers ≥ 6 mmol $g^{-1}$

Die Erfindung wird bevorzugt ausgeführt indem eine Mischung aus M und C in Gegenwart von B polymerisiert wird, wobei für die relativen Massenanteile (bezogen auf die Summe aus M, C und B) gilt:

M: ≤ 56 Gew.%
C: ≥ 40 Gew.%
B: ≥ 4 Gew.%

Insbesondere werden folgende relativen Massenanteile eingesetzt:

M: 30-50 Gew.%
C: 45-70 Gew.%
B: 4-15 Gew.%

In einer besonders bevorzugten Ausführung der Erfindung werden (Meth)acrylsäureester in Gegenwart von Acrylsäure, Methacrylsäure oder Crotonsäure in Emulsionsfahrweise in Gegenwart von polyalkylenoxid-haltigen Silikonderivaten polymerisiert.

Besonders vorteilhaft hat sich die Verwendung von Monomeren M herausgestellt, die als Homopolymerisate eine Glasübergangstemperatur < 25°C aufweisen, insbesondere Ethylacrylat, Methylacrylat, Hydroxyethylacrylat, n-Butylacrylat und Laurylacrylat. Wenn (Meth)acrylate M dieses Typs eingesetzt werden, können Copolymere mit C ≥ 45 Gew.% nahezu ohne Koagulatbildung erhalten werden.

Als geeignete polymerisierbare Monomere M und C werden bevorzugt ethylenisch ungesättigte Monomere verwendet. Dabei kann entweder ein einzelnes Monomer oder Kombinationen von zwei oder mehr Monomeren verwendet werden. Mit polymerisierbar ist gemeint, daß die verwendeten Monomere unter Verwendung irgendeiner konventionellen synthetischen Methode polymerisiert werden können.

Beispielsweise können dies Lösungspolymerisation, Emulsionspolymerisation, umgekehrte Emulsionspolymerisation, Suspensionspolymerisation, umgekehrte Suspensionspolymerisation oder Fällungspolymerisation sein, ohne daß die verwendbaren Methoden darauf beschränkt sind. Bei der Lösungspolymerisation kann Wasser, übliche organische Lösungsmittel oder die erfindungsgemäßen Silikonderivate selbst als Lösungsmittel verwendet werden. Das Verfahren in Emulsion ist jedoch bevorzugt.

Monomere die mit einer durch freie Radikale initiierten Reaktion polymerisiert werden können sind bevorzugt. Der Begriff ethylenisch ungesättigt bedeutet, daß die Monomere zumindest eine polymerisierbare Kohlenstoff-Kohlenstoff Doppelbindung besitzen, die mono-, di-, tri-, oder tetrasubstituiert sein kann.

Die bevorzugten ethylenisch ungesättigten Monomere können durch die folgende allgemeine Formel beschrieben werden:

$$X\text{-}C(O)CR^7\text{=}CHR^6$$

wobei X ausgewählt ist aus der Gruppe der Reste -OH, -OM, $-OR^8$, $NH_2$, $-NHR^8$, $N(R^8)_2$ ;

M ist ein Kation ausgewählt aus der Gruppe bestehend aus: Na+, K+, Mg++, Ca++, Zn++, NH4+, Alkylammonium, Dialkylammonium, Trialkylammonium und Tetraalkylammonium;

die Reste $R^8$ können identisch oder verschieden ausgewählt werden aus der Gruppe bestehend aus -H, C1-C40 linear- oder verzweigtkettige Alkylreste, N,N-Dimethylaminoethyl, 2-Hydroxyethyl, 2-Methoxyethyl, 2-Ethoxyethyl, Hydroxypropyl, Methoxypropyl oder Ethoxypropyl.

$R^7$ und $R^6$ sind unabhängig voneinander ausgewählt aus der Gruppe bestehend aus: -H, $C_1$-$C_8$ linear- oder verzweigtkettige Alkylketten, Methoxy, Ethoxy, 2-Hydroxyethoxy, 2-Methoxyethoxy und 2-Ethoxyethyl.

Repräsentative aber nicht limitierende Beispiele von geeigneten Monomeren sind zum Beispiel Acrylsäure und deren Salze, Ester und Amide. Die Salze können von jedem beliebigen nicht toxischen Metall, Ammonium oder substituierten Ammonium-gegenionen abgeleitet sein.

Die Ester können abgeleitet sein von $C_1$-$C_{40}$ linearen, $C_3$-$C_{40}$ verzweigtkettigen, oder $C_3$-$C_{40}$ carbocyclischen Alkoholen, von mehrfachfunktionellen Alkoholen mit 2 bis etwa 8 Hydroxylgruppen wie Ethylenglycol, Hexylenglycol, Glycerin, and 1,2,6-Hexantriol, von Aminoalkoholen oder von Alkoholethern wie Methoxyethanol und Ethoxyethanol oder Polyethylenglykolen.

Ferner eignen sich N,N-Dialkylaminoalkylacrylate- und methacrylate und N-Dialkylaminoalkylacryl- und -methacrylamide der allgemeinen Formel (II)

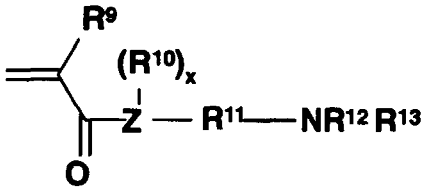

(II)

mit

- $R^9$ H, Alkyl mit 1 bis 8 C-Atomen,
- $R^{10}$ H, Methyl,
- $R^{11}$ Alkylen mit 1 bis 24 C-Atomen, optional substituiert durch Alkyl,
- $R^{12}$, $R^{13}$ $C_1$-$C_{40}$ Alkylrest,
- Z Stickstoff für x = 1 oder Sauerstoff für x = 0.

Die Amide können unsubstituiert, N-Alkyl oder N-alkylamino monosubstituiert, oder N,N-dialkylsubstituiert oder N,N-dialkylamino disubstituiert, worin die Alkyl- oder Alkylaminogruppen von $C_1$-$C_{40}$ linearen, $C_3$-$C_{40}$ verzweigtkettigen, oder $C_3$-$C_{40}$ carbocyclischen Einheiten abgeleitet sind. Zusätzlich können die Alkylaminogruppen quarternisiert werden.

Bevorzugte Monomere der Formel II sind N,N-Dimethylaminomethyl-(meth)acrylat, N,N-Diethylaminomethyl(meth)acrylat, N,N-Dimethylaminoethyl(meth)acrylat, N,N-Diethylaminoethyl(meth)acrylat.

Ebenfalls verwendbare Monomere sind substituierte Acrylsäuren sowie Salze, Ester und Amide davon, wobei die Substituenten an den Kohlenstoffatomen in der zwei oder drei Position der Acrylsäure stehen, und unabhängig voneinander ausgewählt sind aus der Gruppe bestehend aus $C_1$-$C_4$ Alkyl, -CN, COOH besonders bevorzugt Methacrylsäure, Ethacrylsäure und 3-Cyanoacrylsäure. Diese Salze, Ester und Amide dieser substituierten Acrylsäuren können wie oben für die Salze, Ester und Amide der Acrylsäure beschrieben ausgewählt werden.

Andere geeignete Monomere sind Vinyl- und Allylester von $C_1$-$C_{40}$ linearen, $C_3$-$C_{40}$ verzweigtkettigen oder $C_3$-$C_{40}$ carbocyclische Carbonsäuren (z.B.: Vinylacetat, Vinylpropionat, Vinylneononanoat, Vinylneoundekansäure oder t-Butyl-benzoesäure-vinylester); Vinyl- oder Allylhalogenide, bevorzugt Vinylchlorid und Allylchlorid, Vinylether, bevorzugt Methyl-, Ethyl-, Butyl-, oder Dodecylvinylether, Vinylformamid, Vinylmethylacetamid, Vinylamin; Vinyllactame, bevorzugt Vinylpyrrolidon und Vinylcaprolactam, Vinyl- oder Allyl-substituierte heterocyclische Verbindungen, bevorzugt Vinylpyridin, Vinyloxazolin und Allylpyridin.

Weiterhin sind N-Vinylimidazole der allgemeinen Formel III geeignet, worin $R^{14}$ bis $R^{16}$ unabhängig voneinander für Wasserstoff, $C_1$-$C_4$-Alkyl oder Phenyl steht:

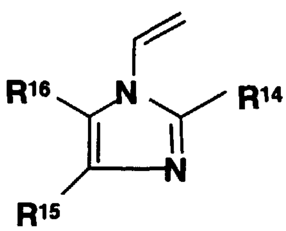

(III)

Weitere geeignete Monomere (a) sind Diallylamine der allgemeinen Formel (IV)

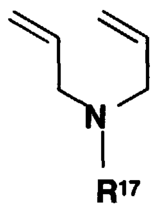

(IV)

mit

$R^{17}$ $C_1$ bis $C_{24}$ Alkyl.

Weitere geeignete Monomere sind Vinylidenchlorid; und Kohlenwasserstoffe mit mindestens einer Kohlenstoff-Kohlenstoff Doppelbindung, bevorzugt Styrol, alpha-Methylstyrol, tert.-Butylstyrol, Butadien, Isopren, Cyclohexadien, Ethylen, Propylen, 1-Buten, 2-Buten, Isobutylen, Vinyltoluol, sowie Mischungen dieser Monomere.

Besonders geeignete Monomere sind Acrylsäure, Methacrylsäure, Ethylacrylsäure, Methylacrylat, Ethylacrylat, Propylacrylat, n-Butylacrylat, iso-Butylacrylat, t-Butylacrylat, 2-Ethylhexylacrylat, Decylacrylat, Methylmethacrylat, Ethylmethacrylat, Propylmethacrylat, n-Butylmethacrylat, iso-Butylmethacrylat, t-Butylmethacrylat, 2-Ethylhexylmethacrylat, Decylmethacrylat, Methylethacrylat, Ethylethacrylat, n-Butylethacrylat, iso-Butylethacrylat, t-Butylethacrylat, 2-Ethylhexylethacrylat, Decylethacrylat, 2,3-Dihydroxypropylacrylat, 2,3-Dihydroxypropylmethacrylat, 2-Hydroxyethylacrylat, Hydroxypropylacrylate, 2-Hydroxyethylmethacrylat, 2-Hydroxyethylethacrylat, 2-Methoxyethylacrylat, 2-Methoxyethylmethacrylat, 2-Methoxyethylethacrylat, 2-Ethoxyethylmethacrylat, 2-Ethoxyethylethacrylat, Hydroxypropylmethacrylate, Glycerylmonoacrylat, Glycerylmonomethacrylat, Polyalkylenglykol(meth)acrylate, ungesättigte Sulfonsäuren wie zum Beispiel Acrylamidopropansulfonsäure; Acrylamid, Methacrylamid, Ethacrylamid, N-Methylacrylamid, N, N-Dimethylacrylamid, N-Ethylacrylamid, N-Isopropylacrylamid, N-Butylacrylamid, N-t-Butylacrylamid, N-Octylacrylamid, N-t-Octylacrylamid, N-Octadecylacrylamid, N-Phenylacrylamid, N-Methylmethacrylamid, N-Ethylmethacrylamid, N-Dodecylmethacrylamid, 1-Vinylimidazol, 1-Vinyl-2-methylimidazol, N,N-Dimethylaminomethyl(meth)acrylat, N,N-Diethylaminomethyl(meth)acrylat, N,N-Dimethylaminoethyl(meth)acrylat, N,N-Diethylaminoethyl(meth)-acrylat, N,N-Dimethylaminobutyl(meth)acrylat, N,N-Diethylaminobutyl(meth)acrylat, N,N-Dimethylaminohexyl(meth)acrylat, N,N-Dimethylaminooctyl(meth)acrylat, N,N-Dimethylaminododecyl(meth)-acrylat, N-[3-(dimethylamino)propyl]methacrylamid, N-[3-(dimethylamino)propyl]acrylamid, N-[3-(dimethylamino)butyl]methacrylamid, N-[8-(dimethylamino)octyl]methacrylamid, N-[12-(dimethylamino)dodecyl]methacrylamid, N-[3-(diethylamino)propyl]-methacrylamid, N-[3-(diethylamino)propyl]acrylamid; Maleinsäure, Fumarsäure, Maleinsäureanhydrid und seine Halbester, Crotonsäure, Itaconsäure, Diallyldimethylammoniumchlorid, Vinylether (zum Beispiel: Methyl-, Ethyl-, Butyl-, oder Dodecylvinylether), Vinylformamid, Vinylmethylacetamid, Vinylamin; Methylvinylketon, Maleimid, Vinylpyridin, Vinylimidazol, Vinylfuran, Styrol, Styrolsulfonat, Allylalkohol, und Mischungen daraus.

Von diesen sind besonders bevorzugt Acrylsäure, Methacrylsäure, Maleinsäure, Fumarsäure, Crotonsäure, Maleinsäureanhydrid sowie dessen Halbester, Methylacrylat, Methylmethacrylat, Ethylacrylat, Ethylmethacrylat, n-Butylacrylat, n-Butylmethacrylat, t-Butylacrylat, t-Butylmethacrylat, Isobutylacrylat, Isobutylmethacrylat, 2-Ethylhexylacrylat, N-t-Butylacrylamid, N-Octylacrylamid, 2-Hydroxyethylacrylat, Hydroxypropylacrylat, 2-Hydroxyethylmethacrylat, Hydroxypropylmethacrylat, Alkylenglykol(meth)-acrylate, ungesättigte Sulfonsäuren wie zum Beispiel Acrylamidopropansulfonsäure, Vinylpyrrolidon, Vinylcaprolactam, Vinylether (z.B.: Methyl-, Ethyl-, Butyl-, oder Dodecylvinylether), Vinylformamid, Vinylmethylacetamid, Vinylamin, 1-Vinylimidazol, 1-Vinyl-2-methylimidazol, N,N-Dimethylaminomethylmethacrylat und N-[3-(dimethylamino)propyl]methacrylamid; 3-Methyl-1-vinylimidazoliumchlorid, 3-Methyl-1-vinylimidazoliummethylsulfat, N,N-Dimethylaminoethylmethacrylat, N-[3-(dimethylamino)propyl]-methacrylamid quaternisiert mit Methylchlorid, Methylsulfat oder Diethylsulfat.

Monomere, mit einem basischen Stickstoffatom, können dabei auf folgende Weise quarternisiert werden: Zur Quaternisierung der Amine eignen sich beispielsweise Alkylhalogenide mit 1 bis 24 C-Atomen in der Alkylgruppe, z.B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid. Weitere geeignete Quaternierungsmittel sind Dialkylsulfate, insbesondere Dimethylsulfat oder Diethylsulfat. Die Quaternierung der basischen Amine kann auch mit Alkylenoxiden wie Ethylenoxid oder Propylenoxid in Gegenwart von Säuren durchgeführt werden. Bevorzugte Quaternierungsmittel sind: Methylchlorid, Dimethylsulfat oder Diethylsulfat.

Die Quaternisierung kann vor der Polymerisation oder nach der Polymerisation durchgeführt werden. Außerdem können die Umsetzungsprodukte von ungesättigten Säuren, wie z.B. Acrylsäure oder Methacrylsäure, mit einem quaternisierten Epichlorhydrin der allgemeinen Formel (V) eingesetzt werden ($R^{18}$ = $C_1$ bis $C_{40}$ Alkyl).

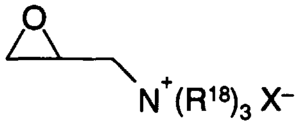

(V)

Beispiele hierfür sind zum Beispiel: (Meth)acryloyloxyhydroxypropyltrimethylammoniumchlorid und (Meth)acryloyloxyhydroxypropyltriethylammoniumchlorid.

Die basischen Monomere können auch kationisiert werden, indem sie mit Mineralsäuren, wie z.B. Schwefelsäure, Chlorwasserstoffsäure, Bromwasserstoffsäure, Iodwasserstoffsäure, Phosphorsäure oder Salpetersäure, oder mit organischen Säuren, wie z.B. Ameisensäure, Essigsäure, Milchsäure, oder Citronensäure, neutralisiert werden.

Zusätzlich zu den oben genannten Monomeren können als Monomere sogenannte Makromonomere wie zum Beispiel silikonhaltige Makromonomere mit ein oder mehreren radikalisch polymerisierbaren Gruppen oder Alkyloxazolinmakromonomere eingesetzt werden wie sie zum Beispiel in der EP 408 311 beschrieben sind.

Des weiteren können fluorhaltige Monomere wie sie beispielsweise in der EP 558423 beschrieben sind, vernetzend wirkende oder das Molekulargewicht regelnde Verbindungen in Kombination oder alleine eingesetzt werden.

Als Regler können die üblichen dem Fachmann bekannten Verbindungen wie zum Beispiel Schwefelverbindungen (z.B.: Mercaptoethanol, 2-Ethylhexylthioglykolat, Thioglykolsäure oder Dodecylmercaptan) sowie Tribromchlormethan oder andere Verbindungen die regelnd auf das Molekulargewicht der erhaltenen Polymerisate wirken, verwendet werden. Es können gegebenenfalls auch thiolgruppenhaltige Silikonverbindungen eingesetzt werden. Bevorzugt werden silikonfreie Regler eingesetzt.

Als vernetzende Monomere können Verbindungen mit mindestens zwei ethylenisch ungesättigten Doppelbindungen eingesetzt werden wie zum Beispiel Ester von ethylenisch ungesättigten Carbonsäuren, wie Acrylsäure oder Methacrylsäure und mehrwertigen Alkoholen, Ether von mindestens zweiwertigen Alkoholen wie zum Beispiel Vinylether oder Allylether. Außerdem geeignet sind geradkettige oder verzweigte, lineare oder cyclische aliphatische oder aromatische Kohlenwasserstoffe, die über mindestens zwei Doppelbindungen verfügen, welche bei den aliphatischen Kohlenwasserstoffen nicht konjugiert sein dürfen. Ferner geeignet sind Amide der Acryl- und Methacrylsäure und N-Allylamine von mindestens zweiwertigen Aminen wie zum Beispiel (1,2-Diaminoethan, 1,3-Diaminopropan). Ferner sind Triallylamin oder entsprechende Ammoniumsalze, N-Vinylverbindungen von Harnstoffderivaten, mindestens zweiwertigen Amiden, Cyanuraten oder Urethanen. Weitere geeignete Vernetzer sind Divinyldioxan, Tetraallylsilan oder Tetravinylsilan.

Besonders bevorzugte Vernetzer sind beispielsweise Methylenbisacrylamid, Triallylamin und Triallylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen die mit Ethylenoxid und/oder Propylenoxid und/oder Epichlorhydrin umgesetzt worden sind.

Geeignete Silikonderivate B sind insbesondere die unter dem Namen Silikontenside oder unter den INCI-Namen Dimethicone Copolyole oder Dimethicone Copolyol Acetate bekannten Verbindungen. Geeignete Silikonderivate B beinhalten Verbindungen mit den CAS-Nummern 64365-23-7; 67762-85-0; 68440-66-4; 68554-65-4; 68937-54-2; 68937-55-3; 68938-54-5; 71965-38-3. Diese sind beispielsweise unter den Markennamen Abil™ (Fa. T. Goldschmidt), Alkasil™ (Fa. Rhône-Poulenc), Silicone Polyol Copolymer™ (Fa. Genesee), Belsil™ (Fa. Wacker), Silwet™ und Silsoft™ (Fa. Witco) oder Dow Corning Silicones DC (Fa. Dow Corning) erhältlich.

Besonders geeignete Silikonderivate B sind solche, die die folgenden Strukturelemente enthalten:

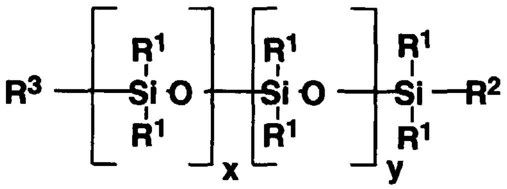 (1)

wobei:

$R^2$ = $CH_3$ oder 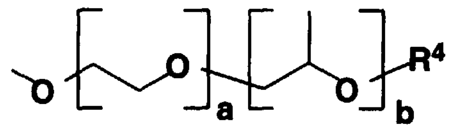

$R^3$ = $CH_3$ oder $R^2$ 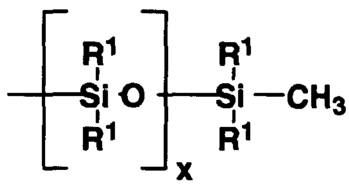

$R^4$ = H, $CH_3$

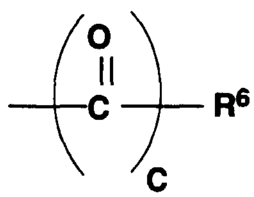

$R^6$ ein organischer Rest aus 1 bis 40 Kohlenstoffatomen, der Amino-, Carbonsäure- oder Sulfonatgruppen enthalten kann oder für den Fall c=O, auch das Anion einer anorganischen Säure bedeutet,

und wobei die Reste $R^1$ identisch oder unterschiedlich sein können, und entweder aus der Gruppe der aliphatischen Kohlenwasserstoffe mit 1 bis 20 Kohlenstoffatomen stammen, cyclische aliphatische Kohlenwasserstoffe mit 3 bis 20 C-Atomen sind, aromatischer Natur oder gleich $R^5$ sind, wobei:

$R^5$ = 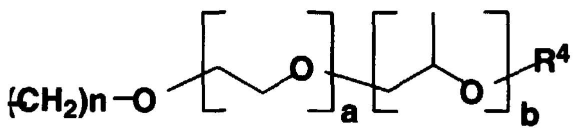

mit der Maßgabe, daß mindestens einer der Reste $R^1$, $R^2$ oder $R^3$ ein polyalkylenoxidhaltiger Rest nach obengenannter Definition ist,

und n eine ganze Zahl von 1 bis 6 ist,

x und y ganze Zahlen derart sind, daß das Molekulargewicht des Polysiloxan-Blocks zwischen 150 und 30000 g/mol liegt,

a,b ganze Zahlen zwischen 0 und 50 sein können mit der Maßgabe, daß die Summe aus a und b größer als 0 ist, und C 0 oder 1 ist.

Bevorzugte Reste $R^2$ und $R^5$ sind solche, bei denen die Summe aus a+b zwischen 5 und 30 beträgt.

Bevorzugt werden die Gruppen $R^1$ aus der folgenden Gruppe ausgewählt: Methyl, Ethyl,Propyl, Butyl, Isobutyl, Pentyl, Isopentyl, Hexyl, Octyl, Decyl, Dodecyl und Octadecyl, cycloaliphatische Reste, speziell Cyclohexyl, aromatische Gruppen, speziell Phenyl oder Naphthyl, gemischt aromatisch-aliphatische Reste wie Benzyl oder Phenylethyl sowie Tolyl und Xylyl und $R^5$.

Besonders geeignete Reste $R^4$ sind solche, bei denen im Falle von $R^4$ = $-(CO)_c-R^6$ $R^6$ ein beliebiger Alkyl-, Cycloalkyl oder Arylrest bedeutet, der zwischen 1 und 40 C-Atomen besitzt und der weitere ionogene Gruppen wie $NH_2$, COOH, $SO_3H$ tragen kann.

Bevorzugte anorganische Reste $R^6$ sind, für den Fall c=O, Phosphat und Sulfat.

Bevorzugte Silikonderivate S sind solche, der allgemeinen Struktur:

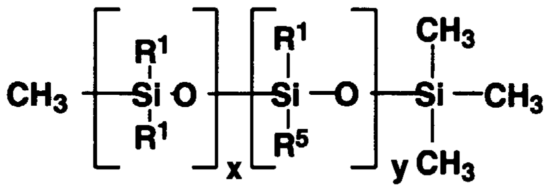

Dabei sind solche Verbindungen besonders bevorzugt, die am Rest $R^5$ als Endgruppe $R^4$ Wasserstoff, n-Butyl, Methyl, oder Acetyl als Rest aufweisen und bei denen $R^1$ Methyl ist.

Bei der Polymerisation der Monomeren A können gegebenenfalls auch andere Polymere wie zum Beispiel Polyamide, Polyurethane, Polyester, Homo- und Copolymere von ethylenisch ungesättigten Monomeren zugegen sein.

Beispiele für solche zum Teil auch in der Kosmetik eingesetzten Polymeren sind die unter den Handelsnamen bekannten Polymere Amerhold™, Ultrahold™, Ultrahold Strong™, Luviflex™ VBM, Luvimer™, Acronal™, Acudyne™, Stepanhold™, Lovocryl™, Versatyl™, Amphomer™ oder Eastman AQ™.

Es können auch andere Polymere nach der Polymerisation den erfindungsgemäßen Polymerzubereitungen beigemischt werden, falls spezielle Eigenschaften eingestellt werden sollen.

Als herkömmliche Polymere eignen sich dazu beispielsweise anionische, kationische, amphotere und neutrale Polymere.

Beispiele für anionische Polymere sind Homo- und Copolymerisate von Acrylsäure und Acrylamid und deren Salze, Natriumsalze von Polyhydroxycarbonsäuren, wasserlösliche oder wasserdispergierbare Polyester, Polyurethane und Polyharnstoffe. Besonders geeignete Polymere sind Copolymere aus tert.-Butylacrylat, Ethylacrylat, Methacrylsäure (z.B. Luvimer™ 100 P), Copolymere aus Ethylacrylat und Methacrylsäure , (z.B. Luvimer™ MAE), Copolymere aus N-tert.-Butylacrylamid, Ethylacrylat, Acrylsäure (Ultrahold Strong™), Copolymere aus Vinylacetat, Crotonsäure und gegebenenfalls weitere Vinylester (z.B. Luviset CA66™, Luviset CAP™ Marken), Maleinsäureanhydridcopolymere, ggf. mit Alkoholen umgesetzt, anionische Polysiloxane, z.B. carboxyfunktionelle Copolymere aus Vinylpyrrolidon, tert.-Butylacrylat, Methacrylsäure (z.B. Luviskol™ VBM) Copolymere von Acrylsäure und Methacrylsäure mit hydrophoben Monomeren, z.B. $C_4$-$C_{30}$-Alkylester der (Meth)acrylsäure, $C_4$-$C_{30}$-Alkylvinylester, $C_4$-$C_{30}$-Alkylvinylether und Hyaluronsäure so wie weitere unter den Handelsnamen bekannte Polymere Amerhold™, Ultrahold™, Luviflex™ VBM 35, Luviset™ P.U.R., Acronal™, Acudyne™, Stepanhold™, Lovocryl™, Versatyl™, Amphomer™(28-4910, LV-71, HC 28-4942), Placise™ L53, Gantrez™ ES 425, Advantage Plus™ (CP, V), Omnirez™ 2000, Resyn ™ 28-1310, Resyn™ 28-2930, Balance™ (0/55, 47), Acudyne™ 255, Aristoflex™ oder Eastman AQ™.

Weitere geeignete Polymere sind kationische Polymere mit der Bezeichnung Polyquaternium nach INCI, z.B. Copolymere aus Vinylpyrrolidon/N-Vinylimidazoliumsalzen (Luviquat™ FC, Luviquat™ HM, Luviquat™ MS, Luviquat™ Care), Copolymere aus N-Vinylpyrrolidon/Dimethylaminoethylmethacrylat, quaternisiert mit Diethylsulfat (Luviquat™ PQ 11), Copolymere aus N-Vinylcaprolactam/N-Vinylpyrrolidon/N-Vinylimidazoliumsalzen (Luviquat™ Hold); kationische Cellulosederivate (Polyquaternium-4 und -10), Acrylamidcopolymere (Polyquaternium-), Styleeze™ CC-10, Aquaflex™ SF-40 und Chitosanderivate.

Als weitere Polymere sind auch neutrale Polymere geeignet wie Polyvinylpyrrolidone, Copolymere aus N-Vinylpyrrolidon und Vinylacetat und/oder Vinylpropionat, Polysiloxane, Polyvinylcaprolactam und Copolymere mit N-Vinylpyrrolidon, Polyethylenimine und deren Salze, Polyvinylamine und deren Salze, Cellulosederivate, Polyasparaginsäuresalze und Derivate. Dazu gehören die unter den folgenden Handelsnamen bekannten Polymer Luviskol™ (K, VA,

Plus), PVP K, PVP/VA Advantage™ HC und H2Old EP-1.

Außerdem geeignet sind auch Biopolymere, d.h. Polymere, die aus natürlich nachwachsenden Rohstoffen gewonnen werden und aus natürlichen Monomerbausteinen aufgebaut sind, z.B. Cellulosederivate, Chitin-, Chitosan-, DANN-, Hyaloronsäure- und RNA-Derivate.

Weitere Polymere sind auch betaine Polymere wie Yukaformer (R205, SM) und Diaformer.

Die erfindungsgemäßen Monomere C können vor oder nach der Polymerisation, zum Teil oder vollständig mit Säuren oder Basen neutralisiert werden um so zum Beispiel die Wasserlöslichkeit oder -dispergierbarkeit auf ein gewünschtes Maß einzustellen.

Als Neutralisationsmittel für Säuregruppen tragende Monomere können zum Beispiel Mineralbasen wie Natriumcarbonat, Alkalihydroxide sowie Ammoniak, organische Basen wie Aminoalkohole speziell 2-Amino-2-Methyl-1-Propanol, Monoethanolamin, Diethanolamin, Triethanolamin, Triisopropanolamin, Tri[(2-hydroxy)1-propyl]amin, 2-Amino-2-Methyl-1,3-Propandiol, 2-Amino-2-hydroxymethyl-1,3-Propandiol sowie Diamine wie zum Beispiel Lysin verwendet werden.

Als Neutralisationsmittel für kationisierbare Gruppen tragende Monomere können zum Beispiel Mineralsäuren wie Salzsäure, Schwefelsäure oder Phosphorsäure, sowie organische Säuren wie Carbonsäuren, Milchsäure, Zitronensäure oder andere eingesetzt werden.

Es können weiterhin Hilfsstoffe wie Weichmacher, Filmbildehilfsmittel, Pigmente, Parfums, Verdicker, Tenside, Konservierungsmittel, kosmetische Wirkstoffe wie Phytantriol, Vitamine und Provitamine, beispielsweise Vitamin A, E und C, Retinol, Bisabolol, Pnthenol, natürliche und synthetische Lichtschutzmittel Naturstoffe, Treibungsmittel, Lösungsvermittler, Repellents, Bleichmittel, Färbemittel, Tönungsmittel, Bräunungsmittel, Reflektoren, Proteine, Ceramid, AHA-Säuren, Fruchtsäuren, Collagen, Eiweißhydrolysate, Stabilisatoren, pH-Wert Regulatoren, Emulgatoren, Gelbildner, Konsistenzgeber, Silikone, Feuchthaltemittel, Rückfetter oder andere übliche Additive, allein oder in Kombination, bei der Polymerisation anwesend sein und/oder nach der Polymerisation zugefügt werden.

Weiterhin können auch keimhemmende Mittel eingesetzt werden. Dazu gehören generell alle geeigneten Konservierungsmittel mit spezifischer Wirkung gegen grampositive Bakterien, z.B. Triclosan (2,4,4'-Trichlor-2'-hydroxydiphenylether), Chlorhexidin (1,1'-Hexamethylenbis[5-(4-chlorphenyl)-biguanid) sowie TTC (3,4,4'-Trichlorcarbanilid). Quartäre Ammonium-Verbindungen sind prinzipiell ebenfalls geeignet, werden jedoch bevorzugt für desinfizierende Seifen und Waschlotionen verwendet. Auch zahlreiche Riechstoffe haben antimikrobielle Eigenschaften. Spezielle Kombinationen mit besonderer Wirksamkeit gegenüber grampositiven Bakterien werden für die Komposition sog. Deoparfums eingesetzt. Auch eine große Anzahl etherischer Öle bzw. deren charakteristische Inhaltsstoffe wie z.B. Nelkenöl (Eugenol), Minzöl (Menthol) oder Thymianöl (Thymol), zeigen eine ausgeprägte antimikrobielle Wirksamkeit.

Die antibakteriell wirksamen Stoffe werden in Konzentrationen von ca. 0.1-0.3 Gew.-% eingesetzt.

Ein weiterer Gegenstand der Erfindung sind kosmetische, dermatologische, hygienische und/oder pharmazeutische Zusammensetzungen, die die erfindungsgemäßen Polymerisate enthalten. Bevorzugt werden die erfindungsgemäßen Polymere als oder in Beschichtungsmitteln für keratinhaltige Oberflächen, wie Haar, Haut und Nägel verwendet.

Bei Verwendung der erfindungsgemäßen Polymere in der Haarkosmetik, speziell bei der Verwendung als Festiger, ist es vorteilhaft die Glastemperatur der Polymerisate durch geeignete Kombination von ethylenisch ungesättigten Monomeren auf Werte größer 20°C einzustellen.

Besonders geeignete Polymere sind solche, die wasserlöslich sind oder deren Wasserdispergierbarkeit so groß ist, daß sie in einem Lösungsmittelgemisch Wasser:Ethanol = 50:50 (Vol.-%:Vol.-%) in einer Menge von mehr als 0,1 g/l, bevorzugt mehr als 0,2 g/L, löslich sind.

Für den Fall, daß die Polymere aus Monomeren bestehen, die neutralisierbare Reste tragen, sind solche Polymere bevorzugt, die in der neutralisierten Form in einem Lösungsmittelgemisch Wasser:Ethanol = 50:50 (Vol.-%:Vol.-%) in einer Menge von mehr als 0,1 g/l, bevorzugt mehr als 0,2 g/L, löslich sind.

Beispielsweise werden die erfindungsgemäßen Polymerisate in kosmetischen Mitteln zur Reinigung der Haut verwendet. Solche kosmetischen Reinigungsmittel sind ausgewählt aus Stückseifen, wie Toilettenseifen, Kernseifen, Transparentseifen, Luxusseifen, Deoseifen, Cremeseifen, Babyseifen, Hautschutzseifen, Abrasiveseifen und Syndets, flüssigen Seifen, wie pastöse Seifen, Schmierseifen und Waschpasten, und flüssigen Wasch-, Dusch-, und Badepräparaten, wie Waschlotionen, Duschbädern, und -gelen, Schaumbädern, Ölbädern und Scrub-Präparaten, Rasierschäume, -lotionen, -cremes.

Sie eignen sich insbesondere für die Haarkosmetik, vorzugsweise in Zubereitungen wie Haarkuren, Haarlotionen, Haarspülungen, Haaremulsionen, Spitzenfluids, Egalisierungsmittel für Dauerwellen, Hot-Oil-Treatment -Präparate, Conditioner, Festigerlotionen Shampoos, Haarfärbemittel oder Haarsprays, Mittel zur Behandlung von Schuppen und Haarausfall sowie in Haarwuchsmitteln.

Die Hautpflegemittel liegen insbesondere als W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen, Hand- und Arbeitsschutzcremes, Salben und Sonnenschutzcremesvor.

Je nach Anwendungsgebiet können die kosmetischen, hygienischen dermatologischen und/oder pharmazeutischen Zubereitungen als Spray (Pumpspray oder Aerosol), Schaum, Gel, Gelspray, Lotion oder Mousse appliziert werden.

Weiterhin eigenen sie sich für hautkosmetische Zubereitungen wie Gesichtswasser, Gesichtsmasken, Deodorantien und andere kosmetische Lotionen und für die Verwendung in der dekorativen Kosmetik, beispielsweise als Abdeckstift, Theaterfarbe, in Mascara und Lidschatten, Lippenstiften, Kajalstiften, Eyelinern, Rouges und Pudern und Augenbrauenstiften sowie als Mundwasser, Zahnpasta oder für medizinische Hautpräparate.

Außerdem können die erfindungsgemäßen Polymerisate verwendet werden als Nose-Strips zur Porenreinigung, in Antiaknemitteln, Repellents, Rasiermitteln, Haarentfernungsmitteln, Intimpflegemitteln, Fußpflegemitteln sowie in der Babypflege.

Weiterhin sind die erfindungsgemäßen Polymere geeignet als Hilfsmittel in der Pharmazie, bevorzugt als oder in Beschichtungsmittel(n) oder Bindemittel(n) für feste Arzeniformen. Sie können auch in Cremes und als Tablettenüberzugsmittel und Tablettenbindemittel verwendet werden.

Die erfindungsgemäßen Copolymere sind in den kosmetischen, dermatologischen, hygienischen und/oder pharmazeutischen Zubereitungen in einer Menge im Bereich von etwa 0,001 bis 20 Gew.-%, bevorzugt 0,1 bis 10 Gew.-%, bezogen auf das Gesamtgewicht des Mittels enthalten.

Als geeignete Lösungsmittel sind insbesondere zu nennen Wasser und niedrige Monoalkohol oder Polyole mit 1 bis 6 Kohlenstoffatomen und Mischungen davon; bevorzugte Monoalkohole oder Polyole sind Ethanol, i-Propanol, Propylenglycol, Glycerin und Sorbit.

Als weitere übliche Zusätze können enthalten sein Fettkörper, wie mineralische und synthetische Öle, wie z.B. Paraffine, Siliconöle und aliphatische Kohlenwasserstoffe mit mehr als 8 Kohlenstoffatomen, tierische und pflanzliche Öle, wie z.B. Sonnenblumenöl, Kokosöl, Avocadoöl, Olivenöl, Lanolin, oder wachse, Fettsäuren, Fettsäureester, wie z.B. Triglyceride von $C_6$-$C_{30}$-Fettsäuren, Wachsester, wie z.B. Jojobaöl, Fettalkohole, Vaseline, hydriertes Lanolin. Selbstverständlich können auch Mischungen derselben verwendet werden.

Übliche Verdickungsmittel in derartigen Formulierungen sind vernetzte Polyacrylsäuren und deren Derivate, Polysaccharide wie Xanthan-gum, Agar-Agar, Alginate oder Tylosen, Cellulosederivate, z.B. Carboxymethylcellulose oder Hydroxycarboxymethylcellulose, Fettalkohole, Monoglyceride und Fettsäuren, Polyvinylalkohol und Polyvinylpyrrolidon.

Als weitere Zusatzstoffe in den o.g. kosmetischen, dermatologischen, hygienischen und/oder pharmazeutischen Zubereitungen können UV-A- und UV-B-Filtersubstanzen mitverwendet werden. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |

| Nr. | Stoff | CAS-Nr. (=Säure) |
|---|---|---|
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfon-(Sulisobenzonum) und das Natriumsalz | 4065-45-6 |
| 13 | 3-(4'-Methyl)benzyliden-bornan-2-on | 36861-47-9 |
| 14 | 3-Benzylidenbornan-2-on | 15087-24-8 |
| 15 | 1-(4'-Isopropylphenyl)-3-phenylpropan-1,3-dion | 63250-25-9 |
| 16 | 4-Isopropylbenzylsalicylat | 94134-93-7 |
| 17 | 3-Imidazol-4-yl-acrylsäure und ihr Ethylester | 104-98-3 |
| 18 | 2-Cyano-3,3-diphenylacrylsäureethylester | 5232-99-5 |
| 19 | 2-Cyano-3,3-diphenylacrylsäure-2'-ethylhexylester | 6197-30-4 |
| 20 | Menthyl-o-aminobenzoate oder: 5-Methyl-2-(1-methylethyl)-2-aminobenzoate | 134-09-8 |
| 21 | Glyceryl p-aminobenzoat oder: 4-Aminobenzoesäure-1-glyceryl-ester | 136-44-7 |
| 22 | 2,2'-Dihydroxy-4-methoxybenzophenon (Dioxybenzone) | 131-53-3 |
| 23 | 2-Hydroxy-4-methoxy-4-methylbenzophenon (Mexonon) | 1641-17-4 |
| 24 | Triethanolamin Salicylat | 2174-16-5 |
| 25 | Dimethoxyphenylglyoxalsäure oder: 3,4-dimethoxy-phenyl-glyoxal-saures Natrium | 4732-70-1 |
| 26 | 3-(4'Sulfo)benzyliden-bornan-2-on und seine Salze | 56039-58-8 |
| 27 | 4-tert.-Butyl-4'-methoxy-dibenzoylmethan | 70356-09-1 |
| 28 | 2,2',4,4'-Tetrahydroxybenzophenon | 131-55-5 |
| 29 | Benzoesäure-4,4'-[[6-[[4-[[(1,1-dimethylethyl)amino]carbonyl]phenyl]amino]-1,3,5-triazin-2,4-diyl]diimino]bis-,bis(2-ethylhexyl)ester | 154702-15-5 |

| Nr. | Stoff | CAS-Nr. (=Säure) |
|---|---|---|
| 30 | 2-(2H-Benzotriazol-2-yl)-4-methyl-6-[2-methyl-3-[1,3,3,3-tetramethyl-1-[(trimethylsilyl)oxy]disiloxanyl]propyl]-phenol | 155633-54-8 |
| 31 | Dimethicon-diethylbenzalmalonat | 207574-74-1 |
| 32 | Bis[2-hydroxy-5-tert.-octyl-3-(benzotriazol-2-yl)phenyl]methan (Bisoctyltriazon) | 103597-45-1 |
| 33 | 1H-Benzimidazol-4,6-disulfonsäure, 2,2'-(1,4-phenylen)bis-, Di-Natriumsalz (Benzimidazylat) | 180898-37-7 |
| 34 | Phenol, 2,2'-[6-(4-methoxyphenyl)-1,3,5-triazin-2,4-diyl]bis[5-[(2-ethylhexyl)oxy]] (Aniso Triazin) | 187393-00-6 |
| 35 | 2,4,6-Trianilin-(o-carbo-2'-ethylhexyl-1'-oxy)-1,3,5-triazin | 88122-99-0 |

Schließlich sind auch mikronisierte Pigmente wie Titandioxid und Zinkoxid zu nennen.

Die nachfolgenden Beispiele sollen die Erfindung näher erläutern:

Beispiele

Synthese von Polymeren
(Die K-Werte der jeweiligen Polymere wurden bei einer Lösungskonzentration des Polymers von 1 Gew.-% in Ethanol gemessen)

Beispiel S1

Ein Gemisch aus 380 g Wasser, 1 g Natrium-Laurylsulfat, 30,5 g Belsil DMC 6031 (Fa. Wacker, CAS-Nr. 71965-38-3) und 45 g Zulauf 1 wird auf 80°C aufgeheizt. Dann werden 8 g einer 7% igen wäßrigen Natrium-Persulfat Lösung zugegeben. Nach 15 min wird Zulauf 1 während zwei Stunden zudosiert. Es wird zwei Stunden bei 80°C nachpolymerisiert.

| | |
|---|---|
| Zulauf 1: | 181,5 g Wasser |
| | 2 g Natrium-Laurylsulfat |
| | 135 g Ethylacrylat |
| | 140 g Methacrylsäure |
| | 1 g Ethylhexylthioglycolat (EHTG). |

Der K-Wert des Produktes beträgt 51,3. Die Säurezahl ist 387 mg KOH; das entspricht einem IEE von 6,9 mmol $g^{-1}$. Der Feststoffgehalt ist 32,6 Gew.-%. Die Koagulatmenge liegt unter 0.5 Gew.-% bezogen auf Feststoff. Das Polymer wird als Emulsion oder nach Trocknen (ggf. Sprühtrocknen oder Trocknen in Wirbelschicht) als Pulver eingesetzt.

Beispiel S2

Wie Beispiel S1, jedoch mit nur 0.25 g EHTG. Der K-Wert des Produktes beträgt 67.5. IEE von 6,9 mmol $g^{-1}$. Der Feststoffgehalt ist 35,2 Gew.-%. Die Koagulatmenge liegt unter 1 Gew.-% bezogen auf Feststoff.

Beispiel S3

Wie Beispiel S1, jedoch ohne EHTG. Der K-Wert des Produktes beträgt 96. IEE von 6,9 mmol $g^{-1}$. Die Koagulatmenge liegt unter 2 Gew.-% bezogen auf Feststoff.

Beispiel S4

Wie Beispiel S1, jedoch nicht mit Belsil DMC 6031 sondern mit Silwet L 7500 (Fa. Witco; CAS-Nummer 68440-66-4, Siliconpfropfcopolymer mit Polypropylenoxid-Seitenketten). Koagulat < 0.5 Gew.-%. K-Wert 50.5.

Beispiel S5

Wie Beispiel S1, jedoch nicht mit Belsil DMC 6031 sondern mit Silicones DC 5200 (Dimethicone Copolyol der Fa. Dow Corning). Kein Koagulat.

Beispiel S6

Wie Beispiel S1, jedoch nicht mit Belsil DMC 6031, sondern mit Belsil DMC 6032 (Dimethicone Copolyol Acetat der Fa. Wacker-Chemie). Kein Koagulat.

Beispiel S7

Wie Beispiel S1, jedoch nicht mit Belsil DMC 6031, sondern mit Silwet L 7604 (Fa. Witco; CAS-Nummer 68937-54-2, Siliconpfropfcopolymer mit Ethylenoxid-Seitenketten). Kein Koagulat.

Vergleichsbeispiel SV1

Zu einer gerührten Vorlage werden 50 g Zulauf 1 und 3.75 g Zulauf 2 getropft. Die Mischung wird dann auf 78°C erhitzt. Danach werden innerhalb 1.5 h der Rest von Zulauf 1 und innerhalb von 2 h der Rest von Zulauf 2 zugetropft. Die Mischung wird weitere 2 h gerührt. Danach wird Zulauf 3 innerhalb von 15 min. zugetropft und noch 2 h bei 78°C gerührt.

Vorlage: 45 g Belsil DMC 6031, 150 g Ethanol

Zulauf 1: 281 g t-Butylacrylat, 93.8 g Methacrylsäure, 75 g Ethanol

Zulauf 2: 1.5 g t-Butyl-perpivalat (75%ig in Isododecan), 100 g Ethanol

Zulauf 3: 0.5 g t-Butyl-perpivalat, 57.5 g Ethanol

Der K-Wert des Produktes beträgt 39,2. Der Feststoffgehalt ist 50,1 Gew.-%.

Test der Anwendungseigenschaften

Haarspray

Beispiel H1

Rezeptur

4.00 % Polymer aus Beispiel A1

0.40 % Aminomethylpropanol
35.00 % Dimethylether
ad 100 % Ethanol

Vergleichsbeispiel HV1

Rezeptur

4.00 % Polymer Luviflex Soft der Fa. BASF,
Copolymer aus 50% Methacrylsäure und 50% Ethylacrylat
0.40 % Aminomethylpropanol
35.00 % Dimethylether
ad 100 % Ethanol

Vergleichsbeispiel HV2

Rezeptur

4.00 % Polymer aus Vergleichsbeispiel SV1
0.40 % Aminomethylpropanol
35.00 % Dimethylether
ad 100 % Ethanol

Anwendungseigenschaften als Haarspray (Haibseitentest):

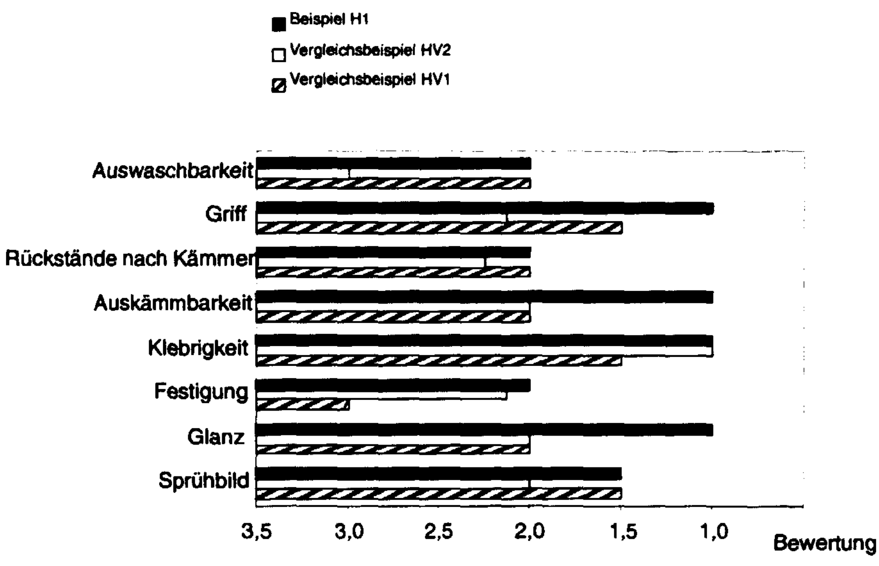

Schaumfestiger

Beispiel M1

Rezeptur

2.00 % Polymer aus Beispiel S1
0.20 % Aminomethylpropanol
2.20 % Luviquat Mono LS (Cocotrimonium methylsulfate) und Parfümöl q.s.
0.10 % Konservierungsmittel
10.00 % Propan/Butan
ad 100.00 % Wasser dest.

Vergleichsbeispiel MV1

Rezeptur

2.00 % Polymer Luviflex Soft der Fa. BASF, Copolymer aus 50% Methacrylsäure und 50% Ethylacrylat
0.20 % Aminomethylpropanol
2.20 % Luviquat Mono LS (Cocotrimonium methylsulfate) und Parfümöl q.s.
0.10 % Konservierungsmittel
10.00 % Propan/Butan
ad 100.00 % Wasser dest.

Anwendungseigenschaften als Schaumfestiger (Halbseitentest):

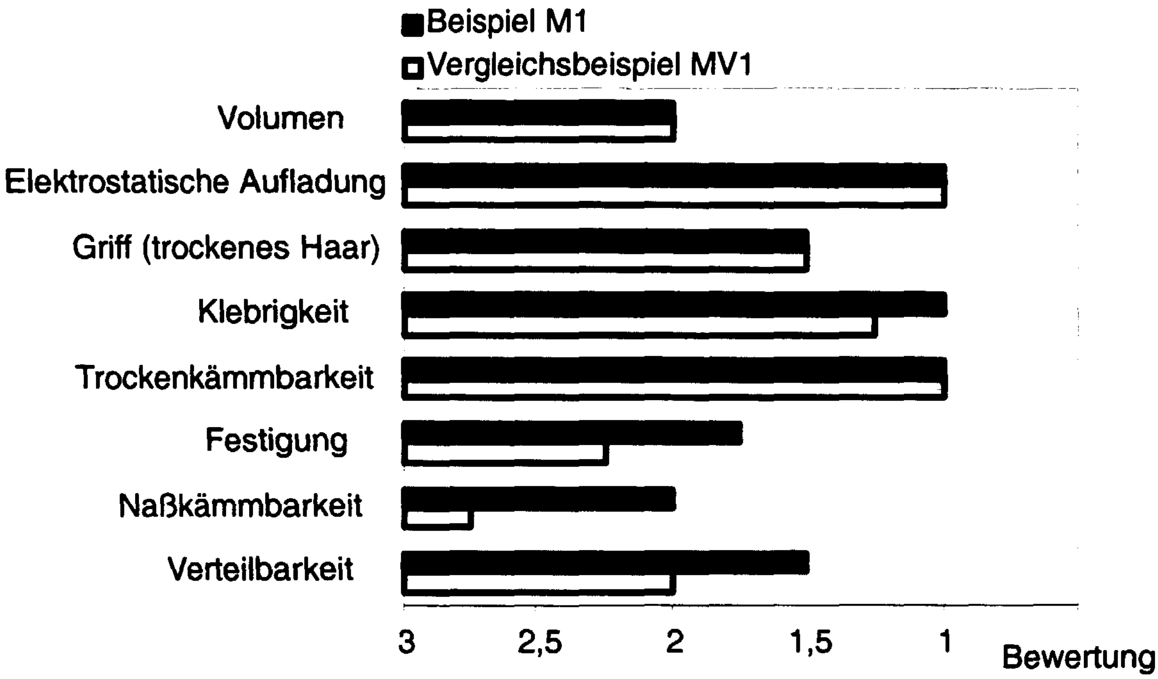

Formulierungen

Beispiel F1: Haarspray

| | |
|---|---|
| Polymer aus Beispiel S1 | 1 g |
| Wasser | ad 100 g |
| Mirapol 550 (CAS-Nr. 26590-05-6) | 3 g |
| Propylenglykol | 2 g |
| Alkamuls EL 719 (Wz. der Fa. Rhodia, CAS-Nr. 61791-12-6) | 2 g |
| Gluadin AGP (Wz. der Fa. Henkel, Hydrolysiertes Weizenprotein, CAS-Nr. 70084-87-6) | 2 g |
| Mirasil DMCO (Wz. der Firma Rhodia, CAS-Nr. 64365-23-7) | 0.5 g |
| UV-Absorber | q. s. |
| Konservierungsstoff | q. s. |
| Parfümöl | q. s. |
| Farbstoff | q. s. |

Beispiel F2: Sonnenschutzgel

| | |
|---|---|
| Polymer aus Beispiel S1 | 1.2 g )[1] |
| Wasser | ad 80.4 g |
| Carbopol 980 (Wz. der Fa. B. F. Goodrich, CTFA-Name Carbomer) | 1.2 g |
| Ethanol, 98%, denatured | 4.0 g |
| Uvinul MS 40 (BASF) | 3.0 g |
| 2-Amino-2-(hydroxymethyl)-1,3-propanediol | 4.0 g |
| Trilon B flüssig (Wz. der Fa. BASF, Tetranatrium-EDTA-Lösung) | 0.3 g |
| Aloe vera gel Konzentrat, 10:1 | 0.5 g |
| d-Panthenol | 0.5 g |
| Parfümöl | 0.2 g |
| Bisabolol | 0.1 g |
| Cremophor A 25 (Wz. der Fa. BASF, CTFA-Bezeichnung Ceteareth-25) | 1.2 g |

)[1] Mengenangabe bezogen auf Festharz

Beispiel F3: Sonnenschutz-Spray

| | |
|---|---|
| Cyclomethicone DC 345 (Wz. der Fa. Dow Corning, cyclisches Oligodimethylsiloxan) | 55.60 |
| Polysynlane (Wz. der Fa. Polyesther, CAS-Nr. 61693-08-1) | 8.00 g |
| Polymer aus Beispiel S1, spühgetrocknet, 50%ig in abs. Ethanol | 3 g |
| Sonnenblumenöl | 3.50 g |
| Vitamin E Acetat | 0.25 g |
| Tenox 6 (Wz. der Fa. Eastman Chemical, Mischung aus Maisöl, Glyceryloleat, Propylenglykol, Phenolderivaten, Propylgallat, Zitronensäure) | 0.15 g |
| Duftstoff | 0.50 g |
| Diisopropyladipat | 5.00 g |
| Octylmethoxycinnamat | 7.50 g |
| Oxybenzone (CAS-Nr. 131-57-7) | 4.00 g |
| Octylsalicylate | 5.00 g |
| Ethoxydiglylol (CAS-Nr. 111-90-0) | 7.50 g |

Beispiel F4: After-Sun Feuchtigkeits-Spray

| | |
|---|---|
| Polymer aus Beispiel S1, wäßrige 36%ige Dispersion | 3.00 g )[1] |
| 2-Amino-2-methyl-1-propanol | 1.50 g |
| Deionized water | ad 84.60 g |
| Luviquat mono CP (Wz. der Fa. BASF, CTFA-Bezeichnung Hydroxyethyl Cetyldimonium Phosphate) | 2.00 g |
| D-panthenol | 0.50 g |
| Propylene glycol | 5.00 g |
| Silicones DC 190 (Wz. der Fa. Dow Corning, CTFA-Bezeichnung Dimethicone Copolyol Acetate) | 0.50 g |
| Prodew 200 (Wz. der Fa. Ajinomoto, Mischung aus Natriumlactat, Pyrrolidoncarbonsäure-Natriumsalz, Sorbitol, Hydrolysiertes Kollagen, Prolin) | 2.00 g |
| Dimethyloldimethylhydantoin (CAS-Nr.6440-58-0) | 0.50 g |
| Cremophor RH 40 (Wz. der Fa. BASF, CAS-Nr. 61788-85-0) | 0.30 g |
| Duftstoff | 0.10 g |

)[1] Mengenangabe bezogen auf Festharz

Beispiel F5: Lippenstift

| | |
|---|---|
| Candelilla (Euphorbia Cerifera) Wachs (CAS-Nr. 8006-44-8) | 4.75 g |
| Bienenwachs | 1.20 g |
| Ozokorit | 7.20 g |
| Polymer aus Beispiel S1, sprühgetrocknet | 3.40 g |
| Microcrystalline Wax SP 96 (Wz. der Fa. Strahl & Pitsch, CAS-Nr. 63231-60-7) | 7.00 g |
| Abil Wax 2440 (Wz. der Fa. Goldschmidt, CTFA-Name Behenoxy Dimethicone) | 3.40 g |
| Isopropyllanolat | 3.40 g |
| Lanolin | 5.75 g |
| Isostearylbehenat | 2.30 g |
| Cetiol LC (Wz. der Fa. Henkel, CTFA-Name Coco-caprylate/caprate) | 12.45 g |
| Limnanthes Alba-Samen Öl | 15.4 g |
| Myristylmyristat | 7.60 g |
| PPG-2 Myristyletherpropionat (CAS-Nr. 74775-06-7) | 9.55 g |
| Micapoly UV Shadow (Wz. der Fa. Centerchem, Mischung aus Mica, Titandioxid, Cyclomethicone, Dimethiconol, Isododecane, Ethylen-Vinylacetat-Copolymer, Eisenoxid) | 3.00 g |
| Lanolin | 4.00 g |
| Perfluorodecalin (CAS-Nr. 306-94-5) | 3.00 g |
| Pigmente | q.s. |

Beispiel F6: Haarpomade

| | |
|---|---|
| Petrolatum (CAS-Nr. 8009-03-8) | 66.2 g |
| Polymer aus Beispiel S1, sprühgetrocknet | 1.4 g |
| Schercemol DID (Wz. der Fa. Scher Chemicals, Diisopropyldilinoleate) | 20.0 g |
| Schercemol BE (Wz. der Fa. Scher Chemicals, Erucasäurebehenylester, CAS-Nr. 18312-32-8) | 9.0 g |
| Cetylalkohol | 4.2 g |
| Propylparaben | 0.1 g |
| Parfümöl | 0.5 g |
| Farbstoffe | q.s. |

Beispiel F7: Haar Mascara

zusammengesetzt aus den Komponenten A, B, C, D

Komponente A

| | |
|---|---|
| Crodafos CES (Wz. der Fa. Croda, Mischung aus Cetearylalkohol, Dicetylphosphat, Ceteth-10 phosphat) | 4.00 g |
| Volpo S-2 (Wz. der Fa. Croda, CTFA-Bezeichnung Steareth-2) | 0.50 g |
| Volpo S-10 (Wz. der Fa. Croda, CTFA-Bezeichnung Steareth-10) | 1.00 g |
| Bienenwachs | 6.50 g |
| Carnaubawachs | 1.25 g |
| Polychol 5 (Wz. der Fa. Croda, CTFA-Bezeichnung Laneth-5) | 0.50 g |
| Stearylalkohol | 1.00 g |

Komponente B

| | |
|---|---|
| Polymer aus Beispiel S1, 36%ige wäßrige Dispersion | 3.00 g )[1] |
| Wasser | ad 57.81 g |
| Polyvinylpyrrolidon K-30 | 1.00 g |
| Natrolsol 250 HHR (Wz. der Fa. Aqualon, Hydroxethylcellulose) | 0.10 g |
| Kaliumhydroxid | 0.19 g |
| $Na_2EDTA$ | 0.10 g |
| Colorona Bordeaux (Wz. der Fa. Rona / E. Merck, Mischung aus Mica und Eisenoxiden) | 12.00 g |
| Propylenglykol | 6.00 g |

)[1] bezogen auf Festharz

Komponente C

| | |
|---|---|
| Hydrotriticum PVP (Wz. der Fa. Croda, Copolymer aus PVP und hydrolysiertem Weizenprotein) | 4.00 g |
| Kaliumhydroxid | 0.05 g |

Komponente D

| | |
|---|---|
| Germaben II (Wz. der Fa. Sutton, Mischung aus Propylenglykol, Diazolidinylharnstoff, Methylharnstoff, Methylparaben und Propylparaben) | 1.00 g |

Beispiel F8: Wäßriger Haarspray

| | |
|---|---|
| Ultrahold Strong (Wz. der Fa. BASF, Copolymer aus Acrylsäure, Ethylacrylat, N-tert.-Butylacrylamid) | 4 g )[1] |
| Luvimer 100 P (Wz. der Fa. Amerchol, Copolymer aus Ethylacrylat, tert.-Butylacrylat und Methacrylsäure) | 0.5 g |
| Polymer aus Beispiel S1 | 3.5 g )[1] |
| 2-Amino-2-methyl-1-propanol | ad pH 9 |
| Wasser | ad 100 g |
| Parfümöl | q. s. |

)[1] Mengenangabe bezogen auf Festharz

Beispiel F9: Alkoholischer Haarspray

| | |
|---|---|
| Polymer aus Beispiel S1, sprühgetrocknet | 3 g |
| 2-Amino-2-methyl-1-propanol | 1.5 g |
| Ethanol | ad 50 g |
| Propan/Butan | ad 100 g |
| Parfümöl | q. s. |

Beispiel F10: Haarspray (Polymerkombination)

| | |
|---|---|
| Polymer aus Beispiel S1, sprühgetrocknet | 1 g |
| 2-Amino-2-methyl-1-propanol | 0.5 g |
| Luviskol VA 37 (Wz. der Fa. BASF, Copolymer aus Vinylpyrrolidon und Vinylacetat) | 8 g |
| Wasser | 5.5 g |
| Ethanol | ad 37.5 g |

(fortgesetzt)

| | |
|---|---|
| Dimethylether | ad 100 g |
| Parfümöl | q. s. |

Beispiel F11: Pflegeschaum

| | |
|---|---|
| Polymer aus Beispiel S1, sprühgetrocknet | 5 g )[1] |
| 2-Amino-2-methyl-1-propanol | 2.5 g |
| Cremophor A 25 (Wz. der Fa. BASF, CTFA-Bezeichnung Ceteareth-25) | 0.2 g |
| Luviquat Mono CP (Wz. der Fa. BASF, CTFA-Bezeichnung Hydroxyethyl Cetyldimonium Phosphate) | 0.5 g |
| Parfümöl | q. s. |
| Konservierungsmittel | q. s. |
| UV-Absorber | q. s. |
| Wasser | ad 90 g |
| Propan/Butan | ad 100 g |

)[1] Mengenangabe bezogen auf Festharz

Beispiel F12: Nagellack (Polymermischung)

| | |
|---|---|
| Polymer aus Beispiel S1, spühgetrocknet | 4 g |
| Gantrez ES-435 (Wz. der Fa. ISP, Copolymer aus Vinylmethylether und und Maleinsäuredibutylester, 50%ige ethanolische Lösung) | 44 g )[1] |
| Rizinusöl | 2 g |
| Ethanol | 47.5 g |
| Diethylphthalat | 2 g |
| 9,10-Anthracenedion (CAS-Nr. 81-48-1) | 0.5 g |
| Parfümöl | q. s. |

)[1] Mengenangabe bezogen auf Festharz

Beispiel F13: Nagellack (Polymermischung)

| | |
|---|---|
| Polymer aus Beispiel S1, spühgetrocknet | 4 g |
| Antaron WP-660 (Wz. der Fa. ISP, Copolymer aus Vinylyrrolidon und $C_{30}$-Olefin) | 44 g |
| Rizinusöl | 2 g |

(fortgesetzt)

| | |
|---|---|
| Ethanol | 47.5 g |
| Diethylphthalat | 2 g |
| 9,10-Anthracenedion (CAS-Nr. 81-48-1) | 0.5 g |
| Parfümöl | q. s. |

**Patentansprüche**

1. Wasserlösliches oder redispergierbares Polymerisat, erhältlich durch radikalische Polymerisation von ethylenisch ungesättigten Monomeren A in Gegenwart wenigstens eines polyalkylenoxid-haltigen Silikonderivates B, dadurch gekennzeichnet, daß für das Produkt aus Ionenaustauscherequivalent IEE und K-Wert des Polymers gilt

$$\text{K-Wert} \times \text{IEE} \geq 150 \text{ mmol g}^{-1},$$

und das IEE > 2.7 mmol $g^{-1}$ ist und, daß das IEE auf Carbonsäuregruppen oder Carboxylatgruppen beruht.

2. Polymerisat gemäß nach Anspruch 1 dadurch gekennzeichnet, daß für das Verhältnis aus Ionenaustauscherequivalent IEE und K-Wert des Polymers und HLB-Wert des Silikonderivats gilt

$$\text{IEE} \times \text{K-Wert / HLB-Wert} \geq 12 \text{ mmol g}^{-1}.$$

3. Polymerisat gemäß Anspruch 1 oder 2 dadurch gekennzeichnet, daß das IEE größer als 3,5 mmol $g^{-1}$ ist.

4. Polymerisat nach einem der Ansprüche 1 bis 3, dadurch gekennzeichnet, daß der K-Wert größer als 45 ist.

5. Polymerisat nach einem der Ansprüch 1 bis 4, dadurch gekennzeichnet, daß der K-Wert des Polymers größer als 45 ist, und daß die ungesättigten Monomere A zu 40-100 Gew.% ungesättigte Carbonsäuren oder deren Salze umfassen.

6. Polymerisat nach einem der Ansprüche 1 bis 5, dadurch gekennzeichnet, daß der HLB-Wert kleiner als 13 ist.

7. Polymerisat nach einem der Ansprüche 1 bis 7, dadurch gekennzeichnet, daß wenigstens zwei ethylenisch ungesättigte Monomere eingesetzt werden, und daß wenigstens eines der beiden Monomere eine ungesättigte Carbonsäure oder deren Salz ist.

8. Polymerisat nach einem der Ansprüche 1 bis 7, dadurch gekennzeichnet, daß 85-96 Gewichtsteile der ungesättigten Monomere A in Gegenwart von 4-15 Gewichtsteilen des Silikonderivats B polymerisiert werden.

9. Polymerisat nach einem der Ansprüche 1 bis 8, dadurch gekennzeichnet, daß die Mengenverhältnisse an

   (a) ethylenisch ungesättigten Monomeren M 30-50 Gew.-%

   (b) ethylenisch ungesättigten Monomeren C mit freien Carboxylatgruppen 45-70 Gew.-% und

   (c) alkylenoxidhaltiger Silikonderivate B 4-15 Gew.-% betragen.

10. Polymerisat nach einem der Ansprüche 1 bis 9, dadurch gekennzeichnet, daß die ungesättigten Monomere A zu 40-100 Gew.% ausgewählt sind aus der Gruppe Acrylsäure, Methacrylsäure, Crotonsäure oder deren Salze.

11. Polymerisat nach einem der Ansprüche 1 bis 10, dadurch gekennzeichnet, daß die ungesättigten Monomere A zu 0,5-60 Gew.% Monomere umfassen, die in homopolymerisierter Form eine Glasübergangstemperatur unter 25°C aufweisen.

12. Polymerisat nach einem der Ansprüche 1 bis 11, dadurch gekennzeichnet, daß die ungesättigten Monomere A zu 0,5-60 Gew.-% ausgewählt sind aus der Gruppe Ethylacrylat, Methylacrylat, Hydroxyethylacrylat, n-Butylacrylat, Lauryl(meth)- acrylat, Stearyl(meth)acrylat.

**13.** Polymerisat nach einem der Ansprüche 1-12, dadurch gekennzeichnet, daß die ungesättigten Monomere A zu 0,5-60 Gew.% ausgewählt sind aus der Gruppe der Verbindungen Methylmethacrylat, tert.-Butylacrylat, Styrol, N-tert.-Butylacrylamid, Vinylacetat, Vinylpropionat, Vinylcaprolactam, Vinylpyrrolidon, Vinylimidazol und N-Methylvinylimidazoliniumsalz.

**14.** Polymerisat nach einem der Ansprüche 1-13, dadurch gekennzeichnet, daß als Silikontensid B Verbindungen der Formel I verwendet werden:

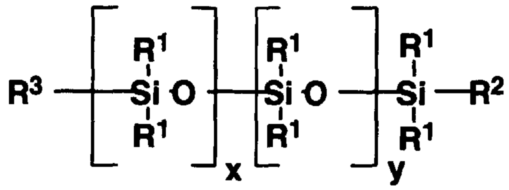

(I)

wobei:

$R^2$ = $CH_3$ oder 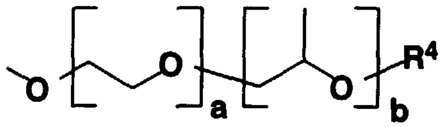

$R^3$ = $CH_3$ oder $R^2$

$R^4$ = H, $CH_3$

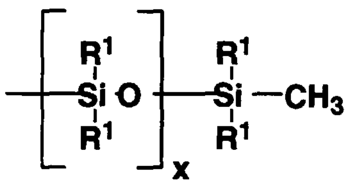

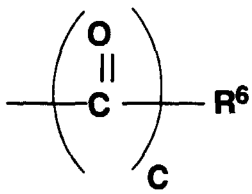

$R^6$ ein organischer Rest aus 1 bis 40 Kohlenstoffatomen, der Amino-, Carbonsäure- oder Sulfonatgruppen enthalten kann oder, für den Fall c=0, auch das Anion einer anorganischen Säure bedeutet,
und wobei die Reste $R^1$ identisch oder unterschiedlich sein können, und entweder aus der Gruppe der aliphatischen Kohlenwasserstoffe mit 1 bis 20 Kohlenstoffatomen stammen, cyclische aliphatische Kohlenwasserstoffe mit 3 bis 20 C-Atomen sind, aromatischer Natur oder gleich $R^5$ sind, wobei:

$R^5$ = 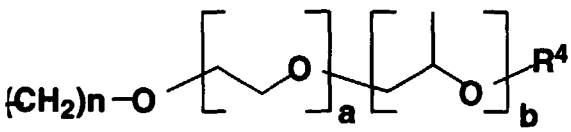

mit der Maßgabe, daß mindestens einer der Reste $R^1$, $R^2$ oder $R^3$ ein polyalkylenoxidhaltiger Rest nach obengenannter Definition ist,
und n eine ganze Zahl von 1 bis 6 ist,

x und y ganze Zahlen derart sind, daß das Molekulargewicht des Polysiloxan-Blocks zwischen 150 und 30000 g/mol liegt,
a und b ganze Zahlen zwischen 0 und 50 sein können mit der Maßgabe, daß die Summe aus a und b größer als 0 ist, und c 0 oder 1 ist.

**15.** Polymerisat gemäß Anspruch 13, dadurch gekennzeichnet, daß Formel I folgende Bedeutung besitzt:

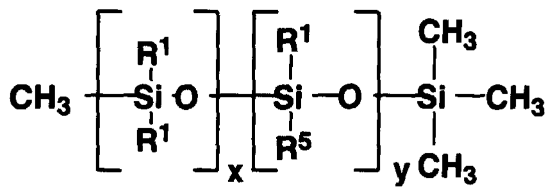

**16.** Verfahren zur Herstellung eines wasserlöslichen oder redispergierbaren Polymerisats, durch radikalische Polymerisation von ethylenisch ungesättigten Monomeren A in Gegenwart wenigstens eines polyalkylenoxid-haltigen Silikonderivates B, dadurch gekennzeichnet, daß für das Produkt aus Ionenaustauscherequivalent IEE und K-Wert des Polymers gilt

$$\text{K-Wert} \times \text{IEE} \geq 150 \text{ mmol g}^{-1},$$

und das IEE > 2.7 mmol $g^{-1}$ ist und, daß das IEE auf Carbonsäuregruppen oder Carboxylatgruppen beruht.

**17.** Verwendung eines Polymerisats nach einem der Ansprüche 1-15, als Zusatzstoff in kosmetischen, dermatologischen, hygienischen und/oder pharmazeutischen Zubereitungen.

**18.** Verwendung des Polymerisats gemäß Anspruch 17, dadurch gekennzeichnet, daß die kosmetischen Zubereitungen ausgewählt sind aus den kosmetischen Mitteln zur Behandlung der Haut und/oder der Haare.

**19.** Verwendung nach Anspruch 18, dadurch gekennzeichnet, daß die kosmetischen Mittel zur Behandlung der Haare ausgewählt sind unter kosmetischen Mitteln zur Pflege und zum Schutz der Haare.

**20.** Verwendung nach Anspruch 19, dadurch gekennzeichnet, daß die kosmetischen Mittel zur Behandlung der Haare ausgewählt sind aus der Gruppe Haarsprays, Schaumfestiger, Haarmousse, Haargel, Festigerelotion, Festiger-Creme, Haarkuren, Haarlotionen, Haarspülungen, Haaremulsionen, Spitzenfluids, Egalisierungsmittel für Dauerwellen, Hot-Oil-Treatment-Präparaten, Conditioner, Festigerlotionen, Shampoos oder Haarfärbemittel.

**21.** Kosmetische, dermatologische, hygienische und/oder pharmazeutische Mittel, enthaltend neben üblichen Zusätzen ein Polymerisat gemäß einem der Ansprüche 1 bis 15.

**22.** Kosmetische, dermatologische, hygienische und/oder pharmazeutische Mittel, enthaltend ein Polymerisat nach einem der Ansprüche 1-15, dadurch gekennzeichnet, daß das Polymerisat in Mischung mit wenigstens einem anderen Polymer und gegebenenfalls in Mischung mit weiteren Stoffen eingesetzt wird.

**23.** Kosmetische, dermatologische, hygienische und/oder pharmazeutische Mittel, enthaltend neben einem Polymerisat gemäß einem der Ansprüche 1 bis 15 ein weiteres anionisches Polymer neben üblichen Zusätzen.

**24.** Kosmetische, dermatologische, hygienische und/oder pharmazeutische Mittel, enthaltend neben einem Polymerisat gemäß einem der Ansprüche 1 bis 15 ein weiteres kationisches Polymer, neben üblichen Zusätzen.

**25.** Kosmetische, dermatologische, hygienische und/oder pharmazeutische Mittel, enthaltend neben einem Polymerisat gemäß einem der Ansprüche 1 bis 15 ein weiteres neutrales Polymer, neben üblichen Zusätzen.

**26.** Kosmetische, dermatologische, hygienische und/oder pharmazeutische Mittel, enthaltend neben einem Polymerisat gemäß einem der Ansprüche 1 bis 15 ein weiteres neutrales Polymer, neben üblichen Zusätzen

**27.** Kosmetische, dermatologische, hygienische und/oder pharmazeutische Mittel, enthaltend neben einem Polymerisat gemäß einem der Ansprüche 1 bis 15 mindestens ein weiteres Biopolymer neben üblichen Zusätzen.

**28.** Verwendung nach Anspruch 17 dadurch gekennzeichnet, daß die Formulierung zu 19 bis 90 Gew.% Wasser enthält.

**29.** Verwendung nach Anspruch 17 dadurch gekennzeichnet, daß die Formulierung wenigstens ein Neutralisationsmittel für das Polymer enthält.

**30.** Verwendung nach Anspruch 17 dadurch gekennzeichnet, daß die Formulierung Ammoniak, organische Basen, 2-Amino-2-methyl-1-propanol, Monoethanolamin, Diethanolamin, Triethanolamin, Triisopropanolamin oder Tri[(2-hydroxy)1-propyl] amin enthält.

**31.** Verwendung nach Anspruch 17 dadurch gekennzeichnet, daß die Formulierung Tenside, grenzflächenaktive Substanzen, UV-Absorber und/oder Verdicker enthält.